# EUROPÄISCHE PATENTANMELDUNG

(11) **EP 2 789 313 A2**
(43) Veröffentlichungstag der Anmeldung: **15.10.2014**
(21) Anmeldenummer: 14158463.1
(22) Anmeldetag: 10.03.2014
(51) Int. Cl.: A61F 2/24

(54) **Einführvorrichtung, insbesondere Katheter, zum Einführen eines medizinischen Hybridimplantats sowie medizinisches Hybridimplantat zu einem Einführen mit einer Einführvorrichtung**

(30) Priorität: 11.04.2013 US 201361810719 P
(71) Anmelder: Biotronik AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft eine Einführvorrichtung (10) zum Einführen eines medizinischen Hybridimplantats (100), wobei das Hybridimplantat (100) zumindest zwei Teilimplantate (102, 104) aufweist, von denen zumindest ein erstes Teilimplantat (102) fremdexpandierbar und zumindest ein zweites Teilimplantat (104) selbstexpandierend ausgeführt ist, aufweisend ein erstes Einführelement (12), das zumindest eine Expansionshilfe (20) zum Expandieren des zumindest ersten fremdexpandierbaren Teilimplantats (102) umfasst, und ein zweites Einführelement (14) und zumindest ein drittes Einführelement (16) zur Freigabe des zumindest zweiten selbstexpandierenden Teilimplantats (104), wobei das zumindest erste fremdexpandierbare Teilimplantat (102) mit der Expansionshilfe (20) expandierbar ist und das zumindest zweite selbstexpandierende Teilimplantat (104) durch eine Relativbewegung zwischen dem zweiten Einführelement (14) und dem zumindest dritten Einführelement (16) freisetzbar ist, wobei das erste Einführelement (12) verschieblich innerhalb des zumindest zweiten Einführelements (14) angeordnet ist.

Die Erfindung betrifft ferner ein Hybridimplantat (100) und ein Verfahren zum Einführen eines solchen Hybridimplantats (100) mit Hilfe der Einführvorrichtung (10).

## Beschreibung

Die Erfindung betrifft eine Einführvorrichtung, insbesondere einen Katheter, zum Einführen eines medizinischen Hybridimplantats zur Implantation in einen tierischen und/oder menschlichen Körper sowie ein Hybridimplantat zu einem Einführen mit einer Einführvorrichtung und ein Verfahren zum Einführen des medizinischen Hybridimplantats mit Hilfe der Einführvorrichtung nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Himschrittmacher für Parkinsonpatienten, Herzimplantate, Cochleaimplantate, Retina Implantate, zahnmedizinische Implantate, Implantate zum Gelenkersatz, Gefäßprotesen oder Stents. Auf dem Feld der Herzimplantate sind beispielsweise Klappenimplantate, wie etwa Aortenklappenimplantate, bekannt, welche die Funktion der natürlichen Aortenklappe übernehmen. Hierbei wird das Klappenimplantat nach Expansion der Implantatstruktur sofort nach der Implantation fixiert und nimmt die Position der natürlichen Aortenklappe ein.

Implantate werden vor einem Einführen in den Körper mit Kathetern verbunden und müssen so befestigt sein, dass sie am Einsatzort komplikationslos vom Katheter genau platziert und definiert freigegeben werden können. Ein häufiges Problem ist hierbei, dass das Implantat mit einer Fehlstellung fixiert wird, was zu einem Versagen des Implantats führen kann. Dies tritt beispielsweise oft bei Kalzifizierung, d.h. die Ablagerung von Kalziumsalzen, insbesondere Kalziumphosphat (Hydroxyapatit) an den Strukturen des Herzens und insbesondere bei stark asymmetrisch kalzifizierte Aortenstenose auf. Um dieser Probleme Herr zu werden, können z.B. Hybridimplantate zum Einsatz kommen, die zwei Einzelimplantate aufweisen, denen unterschiedliche Aufgaben zukommen. Aus US 2011/0166636 A1 ist beispielsweise bekannt, entweder zwei ballonexpandierbare oder zwei selbstexpandierende Stents stufenweise mit einem Katheter zu Implantieren, wobei einer der Stents eine Befestigungsfunktion erfüllt und der andere Stent ein Klappenimplantat aufweist.

Der Erfindung liegt die Aufgabe zugrunde, eine Einführvorrichtung anzugeben, mit der ein hochpräzises und gezieltes Freigeben eines Hybridimplantats erfolgen kann.

Eine weitere Aufgabe ist in der Bereitstellung eines entsprechenden medizinischen Hybridimplantats zu sehen, welches exakt und zuverlässig an einer Implantationsstelle implantiert werden kann.

Eine zudem weitere Aufgabe kann darin gesehen werden, ein Verfahren zum Einführen des erfindungsgemäßen medizinischen Hybridimplantats mit Hilfe der erfindungsgemäßen Einführvorrichtung bereitstellen, bei dem das Einführen und die Freigabe des medizinischen Hybridimplantats, schnell, zuverlässig und komplikationslos erfolgen kann.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine vorgeschlagen Einführvorrichtung zum Einführen eines medizinischen Hybridimplantats vorgeschlagen, wobei das Hybridimplantat zumindest zwei Teilimplantate aufweist, von denen zumindest ein erstes Teilimplantat fremdexpandierbar und zumindest ein zweites Teilimplantat selbstexpandierend ausgeführt ist, aufweisend ein erstes Einführelement, das zumindest eine Expansionshilfe zum Expandieren des zumindest ersten fremdexpandierbaren Teilimplantats umfasst, und ein zweites Einführelement und zumindest ein drittes Einführelement zur Freigabe des zumindest zweiten selbstexpandierenden Teilimplantats, wobei das zumindest erste fremdexpandierbare Teilimplantat mit der Expansionshilfe expandierbar ist und das zumindest zweite selbstexpandierende Teilimplantat durch eine Relativbewegung zwischen dem zweiten und dem zumindest dritten Einführelement freisetzbar ist, wobei das erste Einführelement verschieblich innerhalb des zumindest zweiten Einführelements angeordnet ist.

Durch die erfindungsgemäße Ausgestaltung kann eine Einführvorrichtung, beispielsweise einem Katheter, bereitgestellt werden, mit der das Hybridimplantat besonders schnell und zuverlässig positioniert werden kann. Ebenso kann eine sichere Verankerung des Hybridimplantats am Implantationsort, wie einem Annulus, bereitgestellt werden. Zudem kann mit solch einer Einführvorrichtung ein kompaktes System verwendet werden, da eine radiale Lagerung der Bestandteile der Einführvorrichtung möglich ist und dadurch eine Länge der Einführvorrichtung gegenüber Systemen des Standes der Technik verkürzt werden kann. Ferner weist auch das Hybridimplantat eine geringere Länge im Vergleich zu Implantaten des Standes der Technik auf. Es hat sich gezeigt, dass dadurch auch eine Notwendigkeit eines zusätzlichen stabilisierenden Hilfsapparates am Implantationsbereich, wie einen Herzschrittmacher, verringert werden kann oder besonders vorteilhaft auf diesen verzichtet werden kann.

Des Weiteren können Vorteile von Systemen mit fremdexpandierbaren und selbstexpandierenden Implantaten in einer Einführvorrichtung vereint werden. Dies wäre z.B. eine gute Anpassbarkeit des selbstexpandierenden Teilimplantats an eine Patientenanatomie am Implantationsort, wie beispielsweise einem Annulus einer nativen Herzklappe. Ebenso wäre eine stabile Befestigung mit Hilfe des fremdexpandierbaren Teilimplantats am Implantationsort möglich. Bei herkömmlichen ausschließlich selbstexpandierenden Implantaten muss häufig viel Material, wie beispielsweise Nitinol, eingesetzt werden, um eine ausreichende Stabilität zu gewährleisten. Durch den Einsatz eines erfindungsgemäßen Hybridimplantats könnte eine Metallmenge gegenüber Implantaten des Standes der Technik reduziert werden, was besonders patientenfreundlich ist. Des Weiteren können mit den beiden ungleich expandierbaren Teilimplantaten vorteilhaft unterschiedliche Expansionsmechanismen angewendet werden, was zu einem besonders vielseitig einsetzbaren Hybridimplantat führt.

Besonders vorteilhaft kann mittels der erfindungsgemäßen Ausgestaltungen eine Repositionierung des teilweise freigegebenen Hybridimplantats ermöglicht werden. Dies ist insbesondere der Fall, da das selbstexpandierende Teilimplantat lediglich eine geringe Radialkraft aufweist, die bei einem so genannten "Resheathing" mit wenig Kraftaufwand wieder überwunden werden kann. Zudem kann ein Funktionstest des Hybridimplantats und im Defektfall eine Entfernung des missfunktionalen Hybridimplantats erfolgen. Ein weiterer Vorteil ist, dass bei der Notwenigkeit einer Nachdilatation des Implantationsorts und/oder einem zuführenden Gefäß, wie einer Arterie oder Vene, sich ein Expansionsmittel bzw. ein Ballon zum Weiten schon im Körper befindet. Dies verringert die Strapazen für den Patienten und verkürzt die Zeit des Eingriffs. Da das zweite Einführelement in Bezug auf das zumindest dritte Einführelement axial verschieblich angeordnet, kann das selbstexpandierende Teilimplantat trotz Freilegebewegung des zweiten Einführelements sicher axial fixiert am dritten Einführelement verbleiben.

In diesem Zusammenhang soll unter einem Hybridimplantat ein Implantat aus zumindest zwei Teilimplantaten verstanden werden, wobei die Teilimplantate räumlich und/oder funktional voneinander unabhängig ausgeführt sind. Bevorzugt setzt sich das Hybridimplantat aus zumindest zwei separaten Bauteilen/Teilimplantaten zusammen. Hierbei stellt ein Teilimplantat ein in sich eigenständig funktionierendes und/oder einsetzbares Bauteil dar (Für eine generelle Definition von Implantat wird auf die Ausführungen unten verwiesen.). Unter "fremdexpandierbar" hingegen soll hier "passiv expandierbar" und/oder, dass das Teilimplantat unselbständig und/oder mittels einer von außen zugeführten Kraft expandierbar bzw. plastisch verformbar ist. Konstruktiv einfach kann die passive Expansion mittels einer Expansionshilfe, wie beispielsweise eines mechanischen, pneumatischen und/oder hydraulischen Spreizers oder vorteilhaft eines Ballons erfolgen. Unter "selbstexpandierend" soll hier "aktiv expandierbar" und/oder dass das Teilimplantat selbständig bzw. selbsttätig, also ohne fremde Hilfe expandiert bzw. expandierbar ist verstanden werden. Die Wendung "verschieblich innerhalb des zumindest zweiten Einführelements angeordnet" soll dahingehend verstanden werden, dass das erste Einführelement in einem Lumen des zweiten Einführelements angeordnet ist und relativ zu diesem, insbesondere in axialer Richtung, bewegt werden kann. Hierbei soll unter der axialen Richtung eine Richtung von einem proximalen Ende der Einführrichtung hin zu einem distalen Ende der Einführvorrichtung und/oder umgekehrt verstanden werden.

Das erste fremdexpandierbare Teilimplantat ist bevorzugt auf die Expansionshilfe gecrimpt. Das zweite selbstexpandierende Teilimplantat liegt bevorzugt an einer radialen Innenwand des zweiten Einführelements an, wodurch das zweite Einführelement als ein Klemmkörper für das selbstexpandierende Teilimplantat fungiert. In diesem Zusammenhang soll unter einem "Klemmkörper" ein Körper verstanden werden, der mittels einer Klemmwirkung und/oder eines Kraftschlusses ein anderes Element, insbesondere das Teilimplantat in der Einführvorrichtung in einer festgelegten Position hält. Das selbstexpandierende Teilimplantat wird also in einem Klemmzustand verliersicher in der Einführvorrichtung gehalten. Im Klemmzustand hält eine Wechselwirkung zwischen einer Expansionskraft des Teilimplantats und einer Klemmkraft des zweiten Einführelements des erstere in Position, wodurch ein Entgleiten des Implantats aus der Einführvorrichtung bzw. dem Klemmkörper verhindert wird.

Eine räumlich komfortable Fixierung des selbstexpandierenden Teilimplantats kann bereitgestellt werden, wenn dieses in einer Schutzhülle des zweiten Einführelements fixiert ist. Diese Schutzhülle weist einen größeren radialen Durchmesser als das zweite Einführelement auf und stellt einen funktionalen Teil des zweiten Einführelements dar, d.h. sie kann zusammen mit diesem bewegt werden. Die Schutzhülle ist verliersicher mit dem zweiten Einführelement verbunden. Hierbei kann jede, dem Fachmann für sinnvoll erachtete Verbindungsart, wie ein Kraft-, ein Form- oder ein Stoffschluss, beispielsweise mittels Schweißen, Löten, Schrauben, Nageln oder Kleben, für beide Befestigungsvarianten in Frage kommen. Bei einer Ausgestaltung von zwei verbundenen Bauteilen können Eigenschaften, wie Größe, Material, Beschichtung, Reibung etc., individuell auf die Anforderungen des Bauteils abgestimmt sein. Grundsätzlich kann die Schutzhülle auch einstückig mit dem zweiten Einführelement ausgeführt sein, wobei unter einstückig verstanden werden soll, dass die Schutzhülle und das zweite Einführelement von demselben Bauteil und/oder aus einem Guss gebildet sind und/oder nur unter Funktionsverlust von zumindest einem der Bauteile voneinander getrennt werden können.

Gemäß einer vorteilhaften Realisierung der Erfindung ist das zumindest dritte Einführelement radial zwischen dem ersten Einführelement und dem zweiten Einführelement angeordnet. Hierdurch kann vorteilhaft eine weitere Funktion der Einführvorrichtung realisiert werden. Dies erfolgt beispielsweise indem das zumindest dritte Einführelement dazu ausgebildet ist, zumindest bei einer Expansion des zweiten selbstexpandierenden Teilimplantats dieses in Position zu halten. Dies kann mittels jedem, dem Fachmann für einsetzbar erachteten Prinzip erfolgen, wie beispielweise einem Kraft- und/oder Formschluss, einer Haftreibung oder Ähnlichem. Bevorzugt kann ein distaler Endbereich des zumindest dritten Einführelements als ein Implantathalter ausgeführt sein, der zumindest eine Struktur, wie einen Haken, eine Ösen, einen Schlitze etc., aufweist, der dazu ausgebildet ist, mit einer Kontaktstruktur des Teilimplantats zu interagieren. Diese sind beispielsweise als Ösen ausgeführt, die im montierten Zustand des Teilimplantats in der Einführvorrichtung an dem Ende des Teilimplantats angeordnet sind, das zum proximalen Ende der Einführvorrichtung weist.

Zudem kann die Einführvorrichtung zumindest ein viertes Einführelement umfassen, welches radial innerhalb des ersten Einführelements angeordnet ist, wodurch nochmals eine Funktion integriert werden kann. Unter der Wendung "radial innerhalb des ersten Einführelements angeordnet" soll dahingehend verstanden werden, dass das zumindest vierte Einführelement in einem Lumen des ersten Einführelements angeordnet ist". Zudem kann es verschieblich innerhalb des zweiten Einführelements angeordnet sein bzw. es kann relativ zu diesem, insbesondere in axialer Richtung, bewegt werden. Das zumindest vierte Einführelement ist beispielsweise dazu ausgebildet, ein Führungselement, wie beispielsweise einen Einführungsdraht und/oder einen so genannten "guide wire" aufzunehmen. Damit kann eine Zuführung zum Implantationsort sicher und konstruktiv einfach erfolgen. An einem distalen Ende des Führungselements ist bevorzugt eine Katheterspitze angeordnet.

Somit kann das erste Einführelement als ein Außenschaft (im folgenden Text auch als Ballonaußenschaft bezeichnet) und das vierte Einführelement als ein Innenschaft (im folgenden Text auch als Balloninnenschaft bezeichnet) eines Ballonkatheters sowie das zweite Einführelement als ein Außenschaft (im folgenden Text auch als Katheteraußenschaft bezeichnet) und das dritte Einführelement als ein Innenschaft (im folgenden Text auch als Katheterinnenschaft bezeichnet) eines Katheters, mit dem ein selbstexpandierendes Implantat zugeführt und implantiert werden kann, verstanden werden. Die Einführvorrichtung stellt mit zumindest diesen Komponenten ein Vier-Schaft-Kathetersystem dar. Bei Berücksichtigung eines radial am weitesten außen angeordneten Schaftmantels bzw. eines Stabilisierungsschlauchs kann die Einführvorrichtung als ein Fünf-Schaft-Kathetersystem gesehen werden.

Das erste Einführelement ist unlösbar mit dem zumindest vierten Einführelement verbunden, wodurch diese verliersicher miteinander bewegt werden können. Ist das erste Einführelement in Bezug auf das zumindest dritte Einführelement axial verschieblich angeordnet, kann eine Positionierung der Expansionshilfe mit dem fremdexpandierbaren Teilimplantat relativ zu dem selbstexpandierenden Teilimplantat konstruktiv einfach erfolgen. Die Definition von axial ist analog zu der Definition von axialer Richtung zu verstehen. Ein axialer Bewegungsspielraum für die Expansionshilfe kann vorteilhaft bereitgestellt werden, wenn das zweite Einführelement in Bezug auf das zumindest erste Einführelement angeordnet ist. Auch bleibt eine relative Position der Einführvorrichtung am Implantationsort unbeeinflusst von der Freilegebewegung des zweiten Einführelements, wenn das zweite Einführelement in Bezug auf das zumindest vierte Einführelement axial verschieblich angeordnet ist. Zudem kann eine relative Position der Einführvorrichtung am Implantationsort, sondiert durch das Führungselement mit der Katheterspitze, sicher gehalten werden.

Die Bewegungen können konstruktiv einfach vermittelt werden, wenn das erste Einführelement und/oder das zweite Einführelement und/oder das dritte Einführelement und/oder das zumindest vierte Einführelement jeweils zumindest ein Griffsegment aufweist. Hierbei ist das Griffsegment des ersten und des vierten Einführelements jeweils dazu ausgebildet, in zumindest einem Betriebszustand das erste und das zumindest vierte Einführelement unabhängig von dem zweiten und dem zumindest dritten Einführelement zu bewegen. Da diese Griffsegmente unlösbar miteinander verbunden sind, können beide Einführelemente mit beiden Griffsegmenten bedient werden. Der Betriebszustand kann hier bevorzugt das Positionieren der Expansionshilfe darstellen. Ferner ist das Griffsegment des zweiten Einführelements dazu ausgebildet, in zumindest einem Betriebszustand das zweite Einführelement unabhängig von jeweils dem ersten, dem zumindest dritten und dem zumindest vierten Einführelement zu bewegen. Hier repräsentiert der Betriebszustand insbesondere das Freilegen des zweiten selbstexpandierenden Teilimplantats. Ist eine Bewegung der Einführelemente gegeneinander unerwünscht, können die jeweilig relevanten Einführelemente und/oder deren Griffsegmente lösbar miteinander verbunden werden.

Jedes Griffsegment ist bevorzugt am proximalen Ende der Einführvorrichtung angeordnet bzw. an einem Teil jedes Einführelements, der der sich während der Implantation außerhalb des Körpers befindet. Das Griffsegment kann von einem Gehäuseanteil eines Einführelements und/oder einem separaten, an dem Einführelement angeordneten oder angeformten Bauteil, gebildet sein. Eine gute Bedienbarkeit und ein konstruktiv günstiger Aufbau sind gegeben, wenn das Griffsegment des am weitesten radial innen liegenden Einführelement (Balloninnenschaft) in Richtung des proximalen Endes und/oder am proximalen Ende der Einführvorrichtung und das Griffsegment des am weitesten radial außen liegenden Einführelement (Katheteraußenschaft) in Richtung des distalen Endes der Einführvorrichtung angeordnet ist. Die beiden anderen Griffsegmente liegen jeweils axial dazwischen, das Griffsegment des Ballonaußenschafts mehr in Richtung des proximalen Endes und das Griffsegment des Katheterinnenschafts mehr in Richtung des distalen Endes.

In einer vorteilhaften Ausgestaltung der Erfindung, weist das zumindest dritte Einführelement einen Stopp auf, welcher eine Relativbewegung des zweiten Einführelements bezüglich des zumindest dritten Einführelements limitiert. Hierdurch kann konstruktiv einfach und für einen Bediener intuitiv ein Ausmaß der Bewegung des zweiten Einführelements und damit die Freilegung des selbstexpandierenden Teilimplantats überwacht und/oder eingeschränkt werden. Folglich ist dadurch eine stufenweise Freigabe des selbstexpandierenden Teilimplantats einfach ausführbar. Der Stopp kann hier von jedem, dem Fachmann für realisierbarem Mittel gebildet sein, wie beispielsweise einer (farbige) Markierung, einer Nut, einem Anschlag etc.

Um eine sanfte, reibungs- und störungsarme Bewegung des zweiten Einführelements bei der Freigabe des Hybridimplantats sowie eine komplikationslose Freigabe des Hybridimplantats zu ermöglichen, weist die Einführvorrichtung zudem einen Schaftmantel bzw. einen Stabilisierungsschlauch, welcher sich in Umfangsrichtung um das radial am weitesten außen angeordnete Einführelement und/oder das zweite Einführelement erstreckt. Zur besseren Bedienbarkeit des Schaftmantels bzw. des Stabilisierungsschlauchs und damit der gesamten Einführvorrichtung, weist der Schaftmantel bzw. der Stabilisierungsschlauch in Richtung seines proximalen Endes ein Griffsegment auf. Dieses Griffsegment ist weiter distal angeordnet wie das am weitesten distal angeordnete Griffsegment der Einführelemente, also des Katheteraußenschafts. Der Stabilisierungsschlauch und/oder dessen Griffsegment muss/müssen während der Implantation des Hybridimplantats und insbesondere bei der Bewegung bzw. dem Zurückziehen des zweiten Einführelements in seiner/ihren Position(en) fixiert sein.

Die erfindungsgemäße Einführvorrichtung lässt sich komfortabel einsetzen, wenn im montierten Zustand des Hybridimplantats das zumindest erste fremdexpandierbare Teilimplantat dem distalen Ende der Einführvorrichtung zugewandt ist und/oder das zumindest zweite selbstexpandierende Teilimplantat von dem distalen Ende der Einführvorrichtung abgewandt ist. Mit anderen Worten das fremdexpandierbare Teilimplantat ist distal von dem selbstexpandierenden Teilimplantat angeordnet. Hierdurch kann das fremdexpandierbare Teilimplantat bei der Relativbewegung, insbesondere in Richtung des proximalen Endes der Einführvorrichtung, des ersten Einführelements gegenüber dem zweiten Einführelement in einen Interaktionsbereich des selbstexpandierenden Teilimplantats eintreten (siehe unten). Hierbei stellt der montierte Zustand den Zustand dar, bei welchem beide Teilimplantate an/in der Einführvorrichtung befestigt sind und diese einsatzbereit für eine Implantation des Hybridimplantats ist. Vorteilhafterweise wurde das fremdexpandierbare Teilimplantat bei einer Fabrikation der Einführvorrichtung montiert bzw. auf die Expansionshilfe gecrimpt. Das selbstexpandierende Teilimplantat hingegen wird bevorzugt erst im Vorbereitungslabor in die Einführvorrichtung geladen. Hierdurch kann gewährleistet werden, dass der Klappeneinsatz möglichst zeitnah zur Implantation montiert wird.

Zu einem Eintreten des ersten fremdexpandierbaren Teilimplantats in den Interaktionsbereich des selbstexpandierenden Teilimplantats weist das erste fremdexpandierbare Teilimplantat ebenso zumindest einen Interaktionsbereich zu einer Interaktion mit dem zumindest zweiten selbstexpandierenden Teilimplantat auf, wodurch eine Wechselwirkung der beiden Teilimplantate besonders zuverlässig erfolgen kann.

Gemäße einer vorteilhaften Ausgestaltung umfasst das zumindest zweite selbstexpandierende Teilimplantat zumindest einen Klappeneinsatz, wodurch das Hybridimplantat eine Ersatzfunktion, insbesondere als Rückschlagventil, im Körper übernehmen kann. Ein besonders gut auf seine Ersatzfunktion abgestimmtes Hybridimplantat kann bereitgestellt werden, wenn der zumindest eine Interaktionsbereich des ersten fremdexpandierbaren Teilimplantats infolge einer Relativbewegung zwischen dem ersten Einführelement und dem zweiten Einführelement der Einführvorrichtung distal von einer Klappenebene des Klappeneinsatzes anordenbar ist. Hierdurch kann eine stabile Befestigung des Hybridimplantats am Implantationsort erfolgen, ohne dass der Klappeneinsatz beschädigt und/oder seine Funktion beeinträchtigt wird.

Gemäß einem weiteren Aspekt der Erfindung wird ein medizinisches Hybridimplantat, insbesondere zu einem Einführen mit einer oben beschriebenen Einführvorrichtung, vorgeschlagen. Das medizinische Hybridimplantat weist, wie oben beschrieben, zumindest zwei Teilimplantate auf, von denen zumindest ein erstes Teilimplantat fremdexpandierbar und zumindest ein zweites Teilimplantat selbstexpandierend ausgeführt ist, wobei zumindest ein Interaktionsbereich des ersten fremdexpandierbaren Teilimplantats im expandierten Zustand einen Außendurchmesser und zumindest ein Interaktionsbereich des zumindest zweiten selbstexpandierenden Teilimplantats im expandierten Zustand einen Innenmesser aufweisen, wobei eine Dimension des Außendurchmessers des zumindest einen Interaktionsbereichs des ersten fremdexpandierbaren Teilimplantats an eine Dimension des Innendurchmessers des zumindest einen Interaktionsbereichs des zumindest zweiten selbstexpandierenden Teilimplantats angepasst ist, so dass bei einer Relativbewegung zwischen einem ersten Einführelement und einem zweiten Einführelement der Einführvorrichtung zumindest der eine Interaktionsbereich des ersten fremdexpandierbaren Teilimplantats in den zumindest einen Interaktionsbereich des zumindest zweiten selbstexpandierenden Teilimplantats eintreten kann und der Interaktionsbereich des zweiten selbstexpandierenden Teilimplantats im expandierten Zustand mittels des Interaktionsbereich des ersten fremdexpandierbaren Teilimplantats an einem Bestimmungsort befestigbar ist.

Durch die erfindungsgemäße Ausgestaltung kann ein medizinisches Hybridimplantat bereitgestellt werden, das besonders schnell und zuverlässig positioniert sowie am Implantationsort gute verankert werden kann. Ferner kann es vorteilhaft auf die Parameter bzw. an anatomischen Gegebenheiten des Implantationsorts, wie eine Kalzifizierung einer Blutgefäßwand und/oder eines Annulus, und/oder eine andere, angeborenen und/oder krankhafte Anomalie des Implantationsorts angepasst werden. Des Weiteren weist das Hybridimplantat eine geringere Länge im Vergleich zu Implantaten des Standes der Technik auf. Zudem können kleine French-Größen, beispielsweise zwischen 9 F und 15F, realisiert werden. Des Weiteren können mit den beiden ungleich expandierbaren Teilimplantaten vorteilhaft unterschiedliche Expansionsmechanismen angewendet werden, was zu einem besonders vielseitig einsetzbaren Hybridimplantat führt. Dadurch können Vorteile von beiden Expansionsmechanismen in einem Implantat vereint werden. Dies wäre z.B. eine gute Anpassbarkeit des selbstexpandierenden Teilimplantats an eine Patientenanatomie am Implantationsort, wie beispielsweise einem Annulus einer nativen Herzklappe. Ebenso wäre eine stabile Befestigung mit Hilfe des fremdexpandierbaren Teilimplantats am Implantationsort möglich. Durch den Einsatz eines solchen Hybridimplantats könnte eine Metallmenge gegenüber Implantaten des Standes der Technik reduziert werden, was besonders patientenfreundlich ist. Zudem kann anhand eines Resheathings eine Repositionierung des teilweise freigegebenen Hybridimplantats wegen der geringen Radialkraft des selbstexpandierenden Teilimplantats ermöglicht. Ferner kann ein Funktionstest des Hybridimplantats und im Defektfall eine Entfernung des missfunktionalen Hybridimplantats erfolgen.

In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantate, wie beispielsweise ein Herzimplantat, ein Cochleaimplantat, eine Gefäßprothese, eine Appendixprothese oder besonders vorteilhaft wird eine Ausbildung des medizinischen Hybridimplantats als ein Klappenimplantat vorgeschlagen. Unter einem "Klappenimplantat" soll insbesondere ein Körper verstanden werden, der zumindest eine Ersatzfunktion eines Rückschlagventils, permanent oder für einen längeren Zeitraum bei Implantation erfüllt. Denkbar wären hier sämtliche, dem Fachmann als zweckdienlich erscheinende medizinische Klappenimplantate, wie beispielsweise ein Aortenklappen-, ein Pulmonalklappen-, ein Mitralklappen- oder ein Trikuspidalklappenimplantat.

Besonders vorteilhaft wird eine Ausbildung des medizinischen Hybridimplantats als ein Stent, insbesondere ein Koronarstent, mit einer/einem mit dem Stent reversibel oder irreversibel verbundenen Implantatstruktur und/oder Klappeneinsatz vorgeschlagen. In diesem Zusammenhang soll unter einer "Klappeneinsatz" insbesondere eine Aortenklappe, eine Pulmonalklappe, eine Mitralklappe, eine Trikuspidalklappe oder eine Venenklappe aus natürlichem und/oder künstlichem Material verstanden werden. Besonders bevorzugt ist eine Ausgestaltung des medizinischen Hybridimplantats als Aortenklappe, wodurch eine ausgefeilte Ersatzstruktur für die am häufigsten mit Fehlfunktionen behaftete Herzklappe bereitgestellt werden kann. Auch können Komplikationen wie z.B. Störungen der Mitralklappe oder eine Notwendigkeit eines Herzschrittmachers günstigerweise reduziert werden. Ebenso ist eine Ausgestaltung als Pulmonalklappe oder auch eine Ausgestaltung als Mitralklappe denkbar. Generell wäre jedoch jede andere, dem Fachmann für sinnvoll erscheinende Implantatstruktur denkbar. Durch die Ausführung des Hybridimplantats als Stent kann eine konstruktiv einfach zu implantierende Struktur bereitgestellt werden.

Ferner kann es vorteilhaft sein, wenn das erste fremdexpandierbare Teilimplantat zumindest Kobalt und/oder Chrom aufweist, vorzugsweise in der Form von Edelstahl bzw. medizinischem Edelstahl und/oder eines Cr-Ni-Fe-Stahl - hier bevorzugt die Legierung 316L - oder einem Co-Cr-Stahl. Zudem ist das zweite selbstexpandierende Teilimplantat vorzugsweise aus einem elastischen oder superelastischen Material, etwa einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinendem Material. Möglich wäre auch eine Zink-Kalziumlegierung. Vorteilhafterweise ist das zweite selbstexpandierende Teilimplantat aus einem Formgedächtnis-Material, wie beispielsweise eine Kupfer-Zink-Aluminium-Legierung und/oder Nickel-Titan-Legierung, vorzugsweise Nitinol, gefertigt.

Des Weiteren kann es vorteilhaft sein, wenn das medizinische Hybridimplantat ein Trennmittel aufweist, welches das Material des ersten fremdexpandierbaren Teilimplantat von dem Material des zweiten selbstexpandierende Teilimplantat bzw. deren Interaktionsbereiche trennt. In diesem Zusammenhang soll unter einem "Trennmittel" insbesondere jedes, dem Fachmann für zweckdienlich erscheinendes Mittel, wie ein Abstandshalter und/oder insbesondere eine Beschichtung verstanden werden. Hierbei muss jedoch eine Funktionsübertragung, insbesondere vom fremdexpandierbaren Teilimplantat auf das selbstexpandierende Teilimplantat, gewährleistet sein. unter einer "Beschichtung" soll eine zumindest teilweise und bevorzugt eine vollständige Ummantelung der Interaktionsbereiche bzw. deren Verstrebungen bzw. Stentstruts verstanden werden. Besonders vorteilhaft ist die Beschichtung von einem amorphen Siliziumcarbid gebildet. Generell wäre jedoch jede andere, dem Fachmann für einsetzbar erscheinende Beschichtung denkbar, die ein Inkontaktbringen der Materialien der beiden Teilimplantate und insbesondere der metallischen Materialien, wie insbesondere NiTi oder CoCr, in Gegenwart von Elektrolyten wirkungsvoll verhindert. Durch die Beschichtung kann konstruktiv einfach und Platz sparend ein Kontaktproblem der unterschiedlichen edlen Metalle der beiden Bereiche gelöst werden.

Wie oben beschrieben, umfasst das zumindest zweite selbstexpandierende Teilimplantat zumindest einen Klappeneinsatz, wodurch das Hybridimplantat eine Ersatzfunktion, insbesondere als Rückschlagventil, im Körper übernehmen kann. Da sich das selbstexpandierende Teilimplantat flexibel an die Patientenanatomie anpassen kann, kann sich so auch der Klappeneinsatz vorteilhaft an die Gegebenheiten anpassen. Hierbei kann der Klappeneinsatz mittels jeder, dem Fachmann für sinnvoll erachteter Verbindungsart, wie beispielsweise Nähen und/oder Kleben mit selbstexpandierende Teilimplantat verbunden sein. Wie oben erwähnt, kann das Klappenmaterial künstlichen oder natürlichen Ursprungs (Rind, Schwein, Pferd) sein. Bevorzugt ist die Klappe des Klappeneinsatzes aus Rinder- oder Schweineperikard gefertigt.

Ein besonders gut auf seine Ersatzfunktion abgestimmtes Hybridimplantat kann bereitgestellt werden, wenn der zumindest eine Interaktionsbereich des ersten fremdexpandierbaren Teilimplantats infolge der Relativbewegung zwischen dem ersten Einführelement und dem zweiten Einführelement der Einführvorrichtung distal von der Klappenebene des Klappeneinsatzes anordenbar ist. Hierdurch kann eine stabile Befestigung des Hybridimplantats am Implantationsort erfolgen, ohne dass der Klappeneinsatz beschädigt und/oder seine Funktion beeinträchtigt wird. Damit sieht eine weitere Ausgestaltung der Erfindung vor, dass das fremdexpandierbare Teilimplantat im bestimmungsgemäßen Endzustand in Fließrichtung des Fließmediums axial vor dem Annulus angeordnet ist. Damit kann auch verhindert werden, dass es im Aortenbulbus durch das fremdexpandierbare Teilimplantat zu einem Abdecken der Herzkranzgefäße kommt. Durch diese Realisierung der erfindungsgemäßen Anordnung ist das Hybridimplantat besonders gut an die Anatomie des Herzens bzw. einer Herzklappenregion angepasst. Unter einer "Fließrichtung eines Fließmediums" soll hier insbesondere die wissenschaftlich bekannte Fließrichtung von arteriellem und/oder venösem Blut im Herzen und besonders vorteilhaft im Falle der Aortenklappe der Fluss von Blut vom linken Ventrikel in die Aorta verstanden werden. Hierbei handelt es sich bei dem Annulus bevorzugt um den Aortenannulus.

Gemäß einer weiteren vorteilhaften Ausgestaltung weist das zumindest zweite selbstexpandierende Teilimplantat eine Anzahl von Zellen auf. Diese Zellen können jede, dem Fachmann als zweckdienlich erscheinende Form, wie rund, oval, dreieckig, rechteckig und/oder Rautenform aufweisen. Diese Form ist insbesondere dazu ausgebildet, faltbar zu sein. Besonders bevorzugt sind die Zellen im Wesentlichen rautenförmig ausgestaltet. Unter der Wendung "im Wesentlichen rautenförmig" soll hier verstanden werden, dass auch Formen, die einer Raute bzw. einem Rhombus ähnlich sind, wie beispielsweise eine rautenähnliche Form mit abgerundeten Ecken und/oder konkaven und/oder konvexen Seiten unter dem Begriff "rautenförmig" verstanden werden sollen. Die Zellen stellen das Grundgerüst des Teilimplantats dar. Ferner soll in diesem Zusammenhang unter einem "Grundgerüst" insbesondere eine Struktur, wie beispielsweise ein Drahtgeflecht, verstanden werden, die im Wesentlichen eine Gestalt und/oder eine Form des Teilimplantats vorgibt. Günstigerweise ist die Anzahl der Zellen so ausgewählt, dass das zweite selbstexpandierende Teilimplantat eine geringe Radialkraft aufweist, wodurch ein Resheathing mit minimalem Kraftaufwand möglich ist. Zudem ergibt sich so ein kleiner Crimpdurchmesser des zweiten selbstexpandierenden Teilimplantats. Dadurch kann Bauraum eingespart werden und es kann eine patientenfreundliche Einführvorrichtung mit insbesondere geringen French-Größen zum Einsatz kommen.

Vorteilhafterweise umfasst das zumindest erste fremdexpandierbare Teilimplantat zumindest eine Dichtstruktur, die dazu ausgebildet ist, im implantierten Zustand eine Leckage zu reduzieren. Zusätzlich kann die Dichtstruktur ein Einwachsen des Hybridimplantats fördern. Die Dichtstruktur kann beispielsweise von einem so genannten "textil skirt" gebildet sein, der an einem Teil des fremdexpandierbaren Teilimplantats und insbesondere an einem Teil, der im implantierten Zustand des Hybridimplantats zu dem Klappeneinsatz weist und/oder dem Interaktionsbereich des fremdexpandierbare Teilimplantats, angeordnet ist. Die Dichtstruktur kann beispielsweise aus einem Polyester, wie bspw. Dacron®, gefertigt sein. Die Leckage kann beispielsweise eine valvuläre und/oder eine paravalvuläre Leckage sein.

Beide Teilimplantate können zumindest ein Verankerungsmittel aufweisen, wodurch das Hybridimplantat zusätzlich am Implantationsort befestigt werden kann. In diesem Zusammenhang soll unter einem "Verankerungsmittel" insbesondere eine Schlaufe, ein Haken, eine Spitze und/oder ein anderes, dem Fachmann für anwendbar erachtetes Mittel verstanden werden. Das Verankerungsmittel kann unabhängig von den beiden Teilimplantaten bewegbar ausgeführt sein, wobei unter "bewegbar" hier insbesondere radial bewegbar und besonders vorteilhaft radial bewegbar in Richtung einer Wand eines Blutgefäßes, wie beispielsweise einer Aortenwand, während der Selbstexpansion und/oder Expansion mittels der Expansionshilfe, verstanden werde soll. D.h. weicht der Implantationsort von seiner Form einer erwarteten Kontur ab, kann dies durch die unabhängig bewegbaren Verankerungsmittel ausgeglichen werden. Durch ein Verankerungsmittels kann somit vorteilhaft eine variable Außenkontur des Hybridimplantats im implantierten Zustand eingestellt werden. Zudem kann eine gute Verankerung bei vorteilhaft geringem Kraftaufwand durch die Selbstexpansion bzw. die Expansion mit der Expansionshilfe bereitgestellt werden.

Vorteilhaft kann am Implantat eine ablagerungshemmende, insbesondere kalzifizierungshemmende, Beschichtung vorgesehen sein, insbesondere Homocysteinsäure. Damit kann die Gefahr eine Störung oder ein Funktionsausfall des Hybridimplantats weiter verringert werden.

Zudem wird ein Verfahren zum Einführen eines medizinischen Hybridimplantats mit Hilfe einer Einführvorrichtung vorgeschlagen. Das Verfahren weist zumindest die folgenden Schritte auf: Teilexpandieren eines ersten Teilbereichs eines zumindest zweiten selbstexpandierenden Teilimplantats, insbesondere im auf der Einführvorrichtung montierten Zustand des zumindest zweiten selbstexpandierenden Teilimplantats eines distalen Endbereichs des zumindest zweiten selbstexpandierenden Teilimplantats; Expandieren eines vollständigen zumindest ersten fremdexpandierbare Teilimplantats; Teilexpandieren eines zweiten Teilbereichs des zumindest zweiten selbstexpandierenden Teilimplantats, insbesondere im auf der Einführvorrichtung montierten Zustand des zumindest zweiten selbstexpandierenden Teilimplantats eines proximalen Endbereichs des zumindest zweiten selbstexpandierenden Teilimplantats.

Durch das erfindungsgemäße Verfahren kann das Hybridimplantat besonders schnell und zuverlässig positioniert, verankert sowie implantiert werden. Des Weiteren können mit den beiden ungleich expandierbaren Teilimplantaten vorteilhaft unterschiedliche Expansionsmechanismen angewendet werden, was zu einem besonders vielseitig einsetzbaren Verfahren führt. Dadurch können Vorteile von beiden Expansionsmechanismen in einem Verfahren vereint werden. Besonders vorteilhaft kann ein mittels der erfindungsgemäßen Verfahren mit der Einführvorrichtung verbundenes und schon teilweise freigegebenes Hybridimplantat bedienerfreundlich repositioniert werden. Zudem kann an einem so befestigten Hybridimplantat ein Funktionstest durchgeführt werden und dieses kann im Defektfall entfernt werden. Ein weiterer Vorteil ist, dass bei der Notwenigkeit einer Nachdilatation des Implantationsorts und/oder einem zuführenden Gefäß, wie einer Arterie oder Vene, dies einfach durchgeführt werden kann, da sich das Expansionsmittel bzw. der Ballon zum Weiten schon im Körper befindet. Das erfindungsgemäße Verfahren stellt eine Transkatheter-Aortenklappenimplantations-Methode (Transcatheter Aortic Valve Implantation, TAVI-Methode bzw. Transcatheter Aortic Valve Replacement, TAVR-Methode) für eine Hybrid Bioprothese dar.

Bevorzugt wird das Teilexpandieren des ersten Teilbereichs des zumindest zweiten selbstexpandierenden Teilimplantats durch eine Relativbewegung des zweiten Einführelements bezüglich des dritten Einführelements der Einführvorrichtung ausgelöst, wobei die Relativbewegung anhand eines Stopps des zumindest dritten Einführelements limitiert wird. Hierdurch kann konstruktiv einfach und für einen Bediener intuitiv ein Ausmaß der Bewegung des zweiten Einführelements und damit die Freilegung des selbstexpandierenden Teilimplantats überwacht und/oder eingeschränkt werden. Folglich ist dadurch die stufenweise Freigabe des selbstexpandierenden Teilimplantats einfach ausführbar. Hier erfolgt die Bewegung des zweiten Einführelements insbesondere in Richtung des proximalen Endes der Einführvorrichtung.

Nachfolgend wird in einem Schritt Positionieren die Expansionshilfe mit dem ersten fremdexpandierbaren Teilimplantat teilweise in dem Interaktionsbereich des zweiten selbstexpandierenden Teilimplantats positioniert, so dass das erste fremdexpandierbare Teilimplantat distal von einer Klappenebene des zweiten selbstexpandierenden Teilimplantats angeordnet wird. Dies erfolgt anhand einer Relativbewegung des ersten Einführelements bezüglich des zweiten Einführelements der Einführvorrichtung, wobei die Bewegung des ersten Einführelements insbesondere in Richtung des proximalen Endes der Einführvorrichtung erfolgt.

An dieser Stelle oder auch schon vor dem Positionieren der Expansionshilfe kann eine Positionskontrolle und/oder eine Funktionskontrolle der Klappe durchgeführt werden. Wird eine Fehlposition des ersten Teilbereichs festgestellt oder funktioniert die Klappe ungenügend, kann nun ein Repositionierungsschritt erfolgen. Hierbei wird erst ein Resheathing durchgeführt. Dabei wird das zumindest zweite Einführelement durch eine Relativbewegung bezüglich des ersten Einführelements der Einführvorrichtung erneut über den ersten Teilbereich des zweiten selbstexpandierenden Teilimplantats geschoben, wodurch dieses in seine Ausgangsposition zurück gedrückt wird. Die Bewegung des zweiten Einführelements erfolgt insbesondere in Richtung des distalen Endes der Einführvorrichtung. Nun kann der erste Teilbereich durch erneutes Zurückziehen des zweiten Einführelements positioniert werden.

Vorteilhafterweise wird das Expandieren des ersten fremdexpandierbare Teilimplantats mit Hilfe zumindest einer Expansionshilfe, wie bspw. eines Ballons, durchgeführt, wodurch der erste Teilbereich des zweiten selbstexpandierenden Teilimplantats mittels eines Interaktionsbereichs des ersten fremdexpandierbaren Teilimplantats an einem Implantationsort befestigt wird. Der erste Teilbereich des zweiten selbstexpandierenden Teilimplantats ist auch der Interaktionsbereich dieses Teilimplantats. Dadurch kann der erste Teilbereich des zweiten selbstexpandierenden Teilimplantats zuverlässig an einem Bestimmungsort, wie beispielsweise dem Implantationsort, fixiert werden.

Günstigerweise wird das Teilexpandieren des zweiten Teilbereichs des zumindest zweiten selbstexpandierenden Teilimplantats durch eine Relativbewegung des zweiten Einführelements bezüglich des dritten Einführelements der Einführvorrichtung ausgelöst, wodurch das Hybridimplantat vollständig freigesetzt wird. Damit ist das Hybridimplantat anhand eines schnellen und risikoarmen Verfahrens vollständig implantiert. Die Bewegung des zweiten Einführelements erfolgt insbesondere in Richtung des proximalen Endes der Einführvorrichtung.

Die Erfindung ist nachfolgend beispielhaft, anhand von einem in Zeichnungen dargestellten Ausführungsbeispiel, näher erläutert. Es zeigen in schematischer Darstellung:
- Fig. 1: einen Schnitt durch ein günstiges Ausführungsbeispiel einer erfindungsgemäßen Einführvorrichtung mit einem montierten Hybridimplantat;
- Fig. 2: ein erstes fremdexpandierbares Teilimplantat des Hybridimplantats der Fig. 1 in einer Detaildarstellung;
- Fig. 3: das erstes fremdexpandierbares Teilimplantat der Fig. 2 mit einer schematisch gezeichneten Dichtstruktur;
- Fig. 4: ein zweites selbstexpandierendes Teilimplantat des Hybridimplantats der Fig. 1 in einer Detaildarstellung;
- Fig. 5: die Einführvorrichtung aus Fig. 1 mit platziertem Hybridimplantat zu Beginn einer Implantation des Hybridimplantats;
- Fig. 6: die Einführvorrichtung aus Fig. 1 mit dem teilweise aus einem zweiten Einführelement freigelegten zweiten selbstexpandierenden Teilimplantat der Fig. 4;
- Fig. 7: die Einführvorrichtung aus Fig. 1 mit einer positionierten Expansionshilfe zur Expansion des ersten fremdexpandierbaren Teilimplantats der Fig. 2;
- Fig. 8: die Einführvorrichtung aus Fig. 1 nach der Expansion des ersten fremdexpandierbaren Teilimplantats der Fig. 2 mit Hilfe der Expansionshilfe aus Fig. 7;
- Fig. 9: die Einführvorrichtung aus Fig. 1 nach einer Entleerung der Expansionshilfe;
- Fig. 10: die Einführvorrichtung aus Fig. 1 mit dem komplett aus dem zweiten Einführelement freigelegten zweiten selbstexpandierenden Teilimplantat der Fig. 4;
- Fig. 11: eine Darstellung eines ersten Teils der Verfahrensschritte eines erfindungsgemäßen Einführverfahrens anhand eines Blockdiagramms und;
- Fig. 12: eine Darstellung eines zweiten Teils der Verfahrensschritte des erfindungsgemäßen Einführverfahrens anhand eines Blockdiagramms.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken. Im folgenden Text sind alle distalen Enden der Einführvorrichtung 10 mit dem Bezugszeichen 34, alle distalen Enden der Einführelemente 12, 14, 16, 18 und des Schaftbereichs 36 mit dem Bezugszeichen 42 und alle proximalen Enden der Bauteile der Einführvorrichtung 12, 14, 16, 18, 26, 40 mit dem Bezugszeichen 54 bezeichnet, wobei die mit einem Bindestrich an das jeweilige Bezugszeichen 34, 42, 54 angefügte Zahl beziffert, an welchem Bauteil sich das entsprechende Ende befindet.

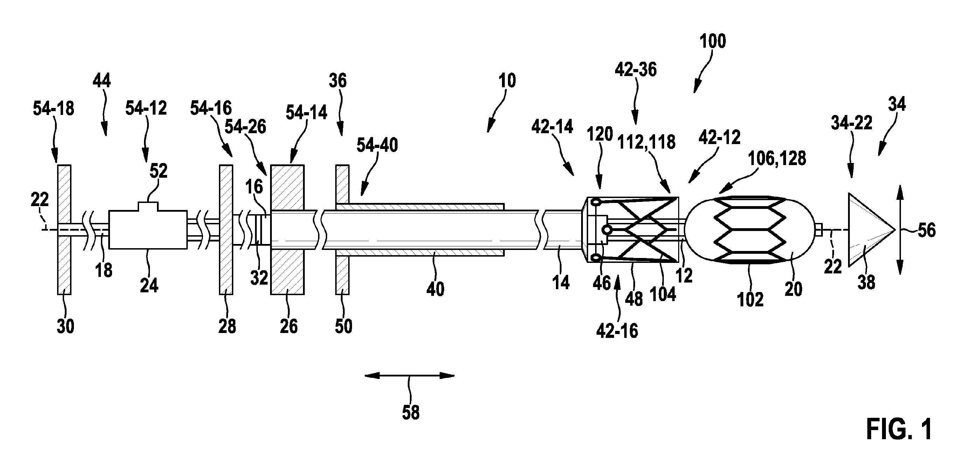

Fig. 1 zeigt einen Längsschnitt durch ein günstiges Ausführungsbeispiel einer Einführvorrichtung 10. Die Einführvorrichtung 10 dient zum Einführen eines medizinischen Hybridimplantats 100 mit zwei Teilimplantaten 102, 104 und ist beispielsweise ein Katheter mit einem Schaftbereich 36 mit vier koaxial angeordneten Einführelementen 12, 14, 16, 18 z.B. einem Balloninnenschaft (viertes Einführelement 18), einem Ballonaußenschaft (erstes Einführelement 12), einem Kathetereinnenschaft (drittes Einführelement 16) und einem Katheteraußenschaft (zweites Einführelement 14). Von diesen vier Einführelementen 12, 14, 16, 18 liegt der zweite Einführelement 14 radial am weitesten außen und umgibt somit die anderen drei Einführelemente 12, 16, 18.

In radialer Richtung 56 nach innen folgt nun das dritte Einführelement 16, das erste Einführelement 12 und am weitesten innen liegt das vierte Einführelement 18. Das dritte Einführelement 16 ist also radial zwischen dem ersten Einführelement 12 und dem zweiten Einführelement 14 und das vierte Einführelement 18 ist radial innerhalb des ersten Einführelements 12 angeordnet. Zudem ist im vierten Einführelement 18 ein Führungselement 22 aufgenommen. Dieses Führungselement 22 ist ein so genannter "guide wire", an dessen distalen Ende 34-22 eine Katheterspitze 38 angeordnet ist. Das Führungselement 22 ist in Teilen in Fig. 1 schematisch als gestrichelte Linie angedeutet. Das zweite, am weitesten außen angeordnete, Einführelement 14 ist wiederum von einem Schaftmantel bzw. einem Stabilisierungsschlauch 40 umgeben. Ferner umfasst das erste Einführelement 12 eine Expansionshilfe 20, in der Form eines Ballons, zum Expandieren des ersten fremdexpandierbaren Teilimplantats 102, wobei die Expansionshilfe 20 an einem distalen Ende 42-12 des ersten Einführelements 12 angeordnet ist. Das zweite Einführelement 14 dient zur Freigabe des zweiten selbstexpandierenden Teilimplantats 104. Dies erfolgt durch eine Relativbewegung in axialer Richtung 58 zwischen dem zweiten Einführelement 14 und dem dritten Einführelement 16. Die vier Einführelemente 12, 14, 16, 18 und der Stabilisierungsschlauch 40 haben folgende Dimensionen:

| Einführelement (12, 14, 16, 18) | ID/OD*mm |
|---|---|
| Lumen Balloninnenschaft | 0,9 / 1,1 |
| Ballonaußenschaft | 1,3 / 1,5 |
| Katheterinnenschaft | 1,7 / 2,0 |
| Katheteraußenschaft | 2,2 / 3,0 (9F) |
| Stabilisierungsschlauch 40 | 3,2 / 4,0 (12F) |

| | |
|---|---|
| *)ID = Innendurchmesser, OD = Außendurchmesser | |

Wie ein Fachkundiger erkennen wird, sind diese Dimensionen im Vergleich zu Vorrichtungen des Standes der Technik deutlich kleiner.

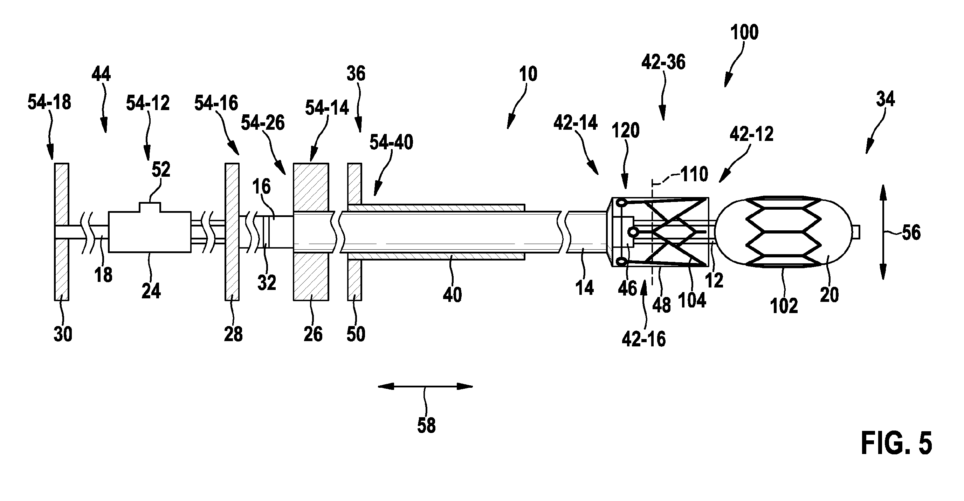

Die Einführvorrichtung 10 ist in Anwenderbetrieb, also während einer Befestigung des Hybridimplantats 100 oder während der Implantation mit ihrem proximalen Ende 44 einem Anwender zugewandt. Das Hybridimplantat 100 ist an einem vom Anwender abgewandten distalen Ende 42-36 des Schaftbereichs 36, beispielsweise in der Nähe der Katheterspitze 38, zwischen dem Balloninnenschaft und dem Katheteraußenschaft platziert und soll am Implantationsort im tierischen oder menschlichen Körper freigegeben werden (vgl. Fig. 5 bis 12).

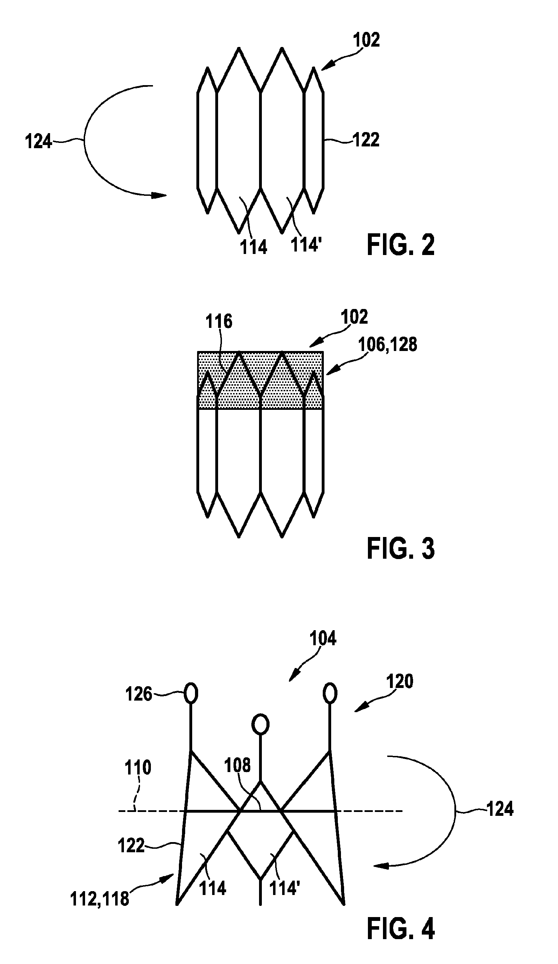

Wie in Fig. 1 zu sehen ist, weist das Hybridimplantat 100 zwei Teilimplantate 102, 104 auf, die in den Fig. 2 und 4 jeweils einzeln gezeigt sind. Das erste Teilimplantat 102 ist fremdexpandierbar ausgeführt und wird im folgenden Text als fremdexpandierbares Teilimplantat 102 benannt (vgl. Fig. 2). Das fremdexpandierbare Teilimplantat 102 weist ein homogen ausgeführtes Grundgerüst 122 mit einer Vielzahl von Zellen 114, 114' auf. Die Zellen 114, 114' sind gleichförmig in Rautenform ausgeführt und erstrecken sich in Umfangsrichtung 124 nebeneinander um einen Umfang des fremdexpandierbaren Teilimplantats 102. Zudem ist das fremdexpandierbare Teilimplantat 102 beispielsweise aus medizinischem Edelstahl oder einer Co-Cr Legierung gefertigt. Während einer Fertigung der Einführvorrichtung 10 wird das fremdexpandierbare Teilimplantat 102 auf die Expansionshilfe 20 der Einfuhrvorrichtung 10 gecrimpt. Hierbei kann beispielsweise eine French-Größe von 15 F oder sogar von 12 F erreicht werden.

Wie in der Fig. 3 gezeigt ist, kann das fremdexpandierbare Teilimplantat 102 eine Dichtstruktur 116 aufweist. Diese ist dazu ausgebildet, im implantierten Zustand eine, insbesondere valvuläre und/oder eine paravalvuläre, Leckage zu reduzieren. Zusätzlich fördert die Dichtstruktur 116 ein Einwachsen des Hybridimplantats 100 am Implantationsort, wie beispielsweise einem Aortenannulus (nicht gezeigt). Im implantierten Zustand des fremdexpandierbaren Teilimplantats 102 ist dieses so angeordnet, dass die Dichtstruktur 116 zu einem Klappeneinsatz 108 des zweiten selbstexpandierenden Teilimplantats 104 weist (siehe unten). Die Dichtstruktur 116 kann beispielsweise von einem so genannten "textil skirt" gebildet sein und ist beispielsweise aus einem Polyester, wie bspw. Dacron®, gefertigt.

Das zweite Teilimplantat 104 ist selbstexpandierend ausgeführt und wird im folgenden Text als selbstexpandierendes Teilimplantat 104 bezeichnet (vgl. Fig. 4). Das selbstexpandierende Teilimplantat 104 ist aus einem Formgedächtnismaterial, wie beispielsweise Nitinol, gefertigt. Zudem weist das selbstexpandierende Teilimplantat 104 ein Grundgerüst 122 mit einer Anzahl von Zellen 114, 114' auf, die so ausgewählt ist, dass das zweite selbstexpandierende Teilimplantat 104 eine geringe Radialkraft hat, hierdurch kann es im Bedarfsfall mit geringem Kraftaufwand wieder zusammengedrückt werden. Die Zellen 114, 114' sind im Wesentlichen rautenförmig und in Umfangsrichtung 124 nebeneinander, jeweils alternierend in der Höhe versetzt angeordnet. Ferner weist das zweite selbstexpandierende Teilimplantat 104 den Klappeneinsatz 108, beispielsweise in der Form einer künstlichen Aortenklappe aus Schweine-Perikard, auf.

An einem Endbereich 120 des selbstexpandierenden Teilimplantats 104, der im auf der Einführvorrichtung 10 montierten Zustand zu dessen proximalem Ende 44 weist, sind in dieser Ausführung drei Befestigungsösen 126 angeordnet und/oder angeformt. Während einer Vorbereitung der Einführvorrichtung 10 im Katheterlabor wird das selbstexpandierende Teilimplantat 104 an einem Implantathalter 46, der sich an einem distalen Ende 42-16 des dritten Einführungselements 16 befindet, befestigt. Hierfür weist der Implantathalter 46 beispielsweise nicht näher gezeigte Haken auf, in welche die Befestigungsösen 126 des selbstexpandierenden Teilimplantats 104 eingreifen (nicht im Detail gezeigt). Dadurch ist das dritte Einführelement 16 dazu ausgebildet, bei einer Expansion des zweiten selbstexpandierenden Teilimplantats 104 dieses in Position zu halten.

Nach der Befestigung des selbstexpandierenden Teilimplantats 104 an dem Implantathalter 46 wird das selbstexpandierende Teilimplantat 104 mit einer Schutzhülle 48 abgedeckt. Diese Schutzhülle 48 ist an einem distalen Ende 42-14 des zweiten Einführungselements 14 angeordnet bzw. angeformt. Die Schutzhülle 48 weist hierfür beispielsweise einen Innendurchmesser (ID) von ca. 4,2 mm und einen Außendurchmesser (OD) von ca. 5,0 mm und damit eine French-Größe von 15 F auf. Generell bestimmen sich diese Dimensionen je nach eingesetztem Hybridimplantat 100 bzw. Klappeneinsatz 108. Das Abdecken erfolgt durch eine Bewegung des zweiten Einführelements 14 in Richtung des distalen Endes 34 der Einführungsvorrichtung 10.

Beide Teilimplantate 102, 104 weisen Interaktionsbereiche 106, 112 auf, die dazu ausgelegt sind, dass die beiden Teilimplantate 102, 104 miteinander interagieren können. Der Interaktionsbereich 106 des fremdexpandierbaren Teilimplantats 102 stellt im auf der Expansionshilfe 20 montierten Zustand des fremdexpandierbaren Teilimplantats 102 dessen proximalen Endbereich 128 dar, der zum proximalen Ende 44 der Einführvorrichtung 10 weist. Am Interaktionsbereich 106 ist zudem die Dichtstruktur 116 angeordnet. Bei dem selbstexpandierenden Teilimplantat 104 befindet sich dessen Interaktionbereich 112 im in der Schutzhülle 48 montierten Zustand an einem distalen Endbereich 118 des selbstexpandierenden Teilimplantats 104, der zum distalen Ende 34 der Einführvorrichtung 10 weist. Somit liegt der Interaktionsbereich 112 axial gegenüber des Endbereichs 120 mit de Befestigungsösen 126.

Wie aus Fig. 1 hervorgeht, weist jedes der Einführelemente 12, 14, 16, 18 und der Stabilisierungsschlauch 40 ein Griffsegment 24, 26, 28, 30, 50 auf, welches jeweils dazu ausgebildet ist, das jeweilige Einführelement 12, 14, 16, 18 und den Stabilisierungsschlauch 40 zu bewegen. Hierbei sind die Griffsegmente 26, 28, 30, 50 der Einführelemente 14, 16, 18 und des Stabilisierungsschlauchs 40 jeweils als sich im Wesentlichen senkrecht zu einer Längsachse des proximalen Anteils der Einführvorrichtung 10 erstreckende Handgriffe ausgeführt. Zudem sind die Griffsegmente 26, 28, 30, 50 jeweils mit einem Luer Lock zum Entlüften des jeweiligen Einführelements 14, 16, 18 bzw. des Stabilisierungsschlauchs 40 ausgestattet (nicht gezeigt). Das Griffsegment 26 ist lösbar mit dem Einführelement 16 oder mit dem Griffsegment 28 und das Griffsegment 28 ist lösbar mit dem Einführelement 12 verbunden (nicht im Detail dargestellt). Dadurch kann beim Einführen der Einführvorrichtung 10 eine relative Bewegung zwischen dem Griffsegment 26 und dem Einführelement 16 sowie zwischen dem Griffsegment 28 und dem Einführelement 12 verhindert werden. Das Griffsegment 50 dient zum Fixieren des Stabilisierungsschlauchs 40.

Das Griffsegment 24 des ersten Einführelements 12 hingegen ist von einer Weiche 52 zur Inflation bzw. Deflation der Expansionshilfe 20 gebildet (siehe unten). Die Griffsegmente 24, 30 sind unlösbar miteinander verbunden, wodurch die Einführelemente 12, 18 sowohl mit dem Griffsegment 24 als auch mir Griffsegment 30 zusammen bewegt werden können. Alle Griffsegmente 24, 26, 28, 30, 50 sind jeweils an einem proximalen Ende 54-12, 54-14, 54-16, 54-18, 54-40 des jeweiligen Einführelements 12, 14, 16, 18 und des Stabilisierungsschlauchs 40 angeordnet. Zusätzlich können die proximalen Schaftbereiche der Einführelemente 12, 14, 16, 18 ebenso als Griffsegment benutzt werden.

Das zumindest dritte Einführelement 16 weist zudem einen Stopp 32 auf, welcher eine Relativbewegung des zweiten Einführelements 14 bezüglich des dritten Einführelements 16 limitiert. Dieser Stopp 32 ist beispielsweise von einer Markierung gebildet.

Im montierten Zustand des Hybridimplantats 100 in/an der Einführvorrichtung 10, wie dies in der Fig. 1 und 5 zu sehen ist, ist das erste fremdexpandierbare Teilimplantat 102 dem distalen Ende 34 der Einführvorrichtung 10 zugewandt und das zweite selbstexpandierende Teilimplantat 104 ist von dem distalen Ende 34 der Einführvorrichtung 10 abgewandt. Mit anderen Worten, das fremdexpandierbare Teilimplantat 102 ist distal vom selbstexpandierenden Teilimplantat 104 angeordnet. Die Interaktionsbereich 106, 112 weisen also zueinander.

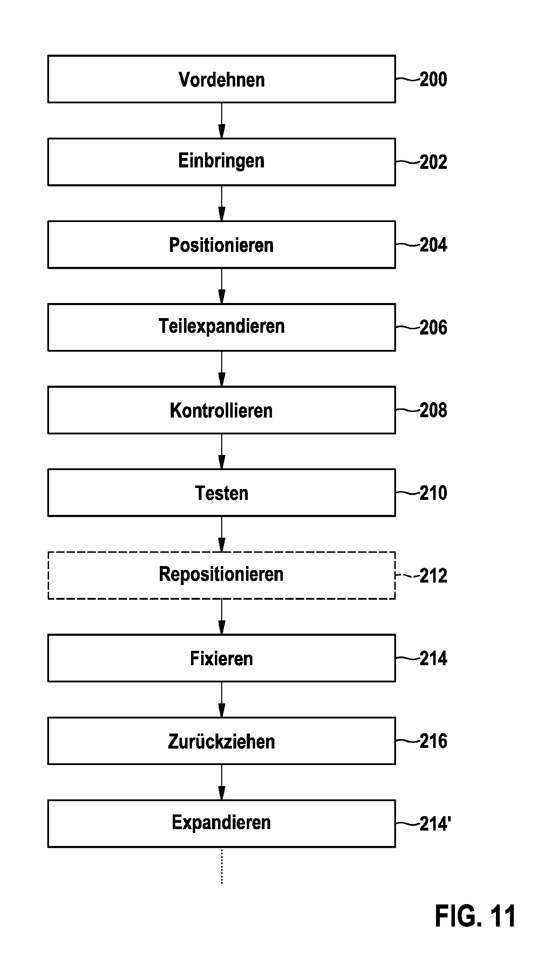

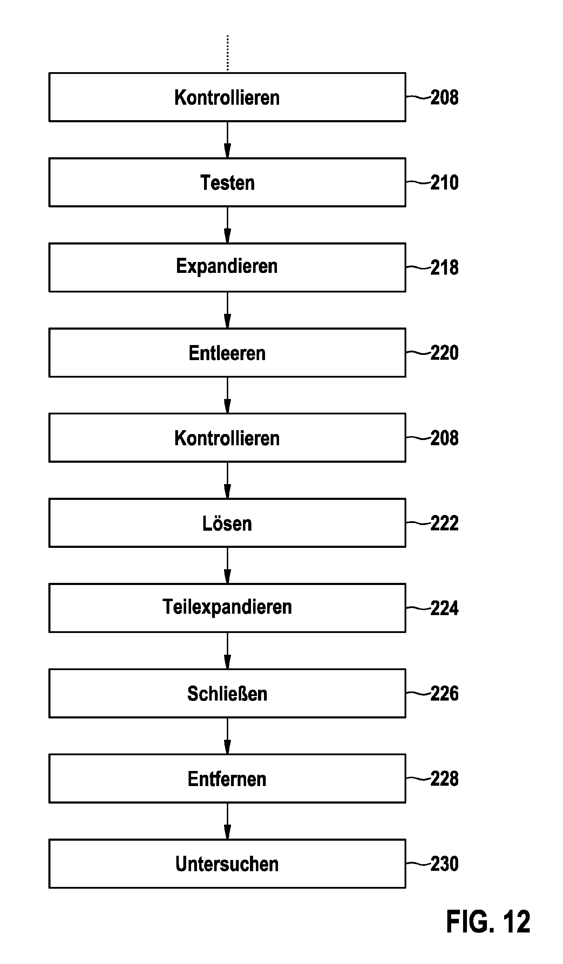

Im Folgenden ist ein Verfahren zum Einführen eines medizinischen Hybridimplantats 100 mit Hilfe der Einführvorrichtung 10 anhand der Fig. 5 bis 10 und der Blockdiagramme der Fig. 11 und 12 beschrieben. Die beiden Teilimplantate 102, 104 sind, wie oben beschrieben und in Fig. 5 gezeigt ist, in/an der Einführvorrichtung 10 montiert (siehe Fig. 5).

Vor dem Einführen der Einführvorrichtung 10 wird die native Aortenklappe in Schritt 200 Vordehnen mit einem so genannten Valvuloplastie-Ballon vorgedehnt. Falls das Führungselement 22 noch nicht in der Einführvorrichtung 10 platziert ist, wird diese nun in Schritt 202 Einbringen über das Führungselement 22 geschoben. Anschließend wird die so vorbereitete Einführvorrichtung 10 zu einer Implantation des Hybridimplantats 100 im Körper in einer bekannten Weise in den Körper eingeführt und in Schritt 204 Positionieren positioniert. Hierbei sollte eine Klappenebene 110 des Klappeneinsatzes 108 bündig mit dem Annulus der natürlichen Klappe angeordnet sein (nicht gezeigt).

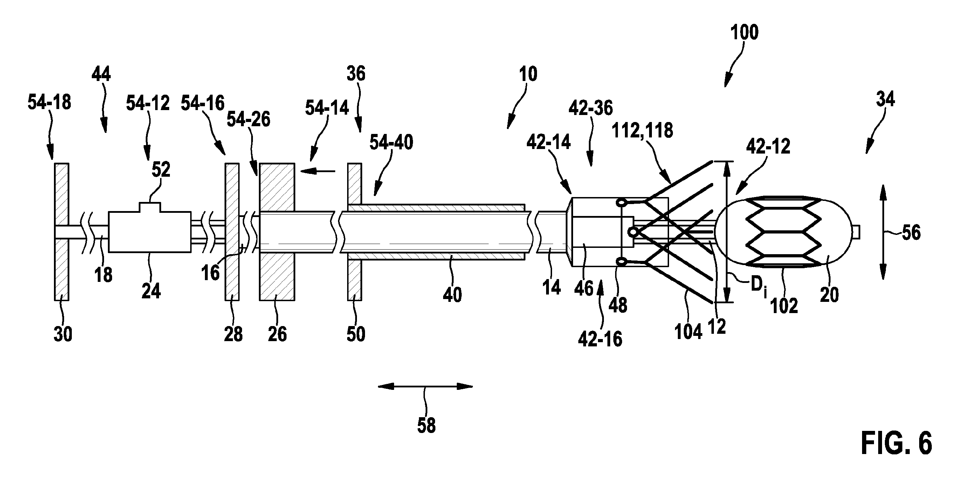

Nun beginnt ein Platzieren des Hybridimplantats 100. Dabei wird in einem ersten Schritt 206 Teilexpandieren ein erster Teilbereich 118 bzw. der distale Endbereich 118 des selbstexpandierenden Teilimplantats 104 freigelegt, wodurch dieser selbsttätig expandiert (vgl. Fig. 6). Hierbei wird nach einem Lösen der Verbindung zwischen dem Griffsegment 26 und dem Einführelement 16 oder mit dem Griffsegment 28 das zweite Einführelement 14 mit dem Griffsegment 26 in Richtung des proximalen Endes 44 der Einführvorrichtung 10 gezogen (siehe Pfeil), wodurch das zweite Einführelement 14 eine Relativbewegung in axialer Richtung 58 bezüglich der anderen drei Einführelemente 12, 16, 18 vollzieht. Das Teilexpandieren des ersten Teilbereichs 118 wird somit durch eine Relativbewegung des zweiten Einführelements 14 bezüglich des dritten Einführelements 16 ausgelöst. Die Ziehbewegung erfolgt hierbei so lange, bis sich ein proximales Ende 54-26 des Griffsegments 26 des zweiten Einführelements 14 auf einer axialen Höhe des Stopps 32 des dritten Einführelements 16 befindet (siehe die axialen Höhen des Griffsegments 26 der Fig. 5 und 6). Somit wird die Relativbewegung anhand des Stopps 32 limitiert (siehe Fig. 6).

Damit ist das selbstexpandierende Teilimplantats 104 teilweise freigelegt und der Interaktionsbereich 112 hat in seinem so expandierten Zustand einen Innendurchmessers Dᵢ angenommen, der in seiner Dimension an eine Dimension eines Außendurchmessers Dₐ des Interaktionsbereichs 106 des ersten fremdexpandierbaren Teilimplantats 102 in dessen expandiertem Zustand angepasst ist. Hierbei ist der der Innendurchmesser Dᵢ des selbstexpandierenden Teilimplantats 104 minimal größer wie der Außendurchmesser Dₐ des fremdexpandierbaren Teilimplantats 102, so dass dieses in den Innendurchmesser Dᵢ des selbstexpandierenden Teilimplantats 104 eintreten kann (siehe unten).

An dieser Stelle kann in Schritt 208 Kontrollieren eine Positionskontrolle des expandierten Teilbereichs 118 durchgeführt werden. Zusätzlich können nun in Schritt 210 Testen Funktionstests des Hybridimplantats 100 oder des Klappeneinsatzes 108 ausgeführt werden. Wird eine Fehlposition des ersten Teilbereichs 118 festgestellt oder funktioniert der Klappeneinsatz 108 ungenügend, kann nun in Schritt 212 Repositionieren eine Repositionierung erfolgen. Hierbei wird erst ein Resheathing durchgeführt. Dabei wird das zweite Einführelement 14 durch eine Relativbewegung in axialer Richtung 58 bezüglich des dritten Einführelements 16 erneut über den ersten Teilbereich 118 des zweiten selbstexpandierenden Teilimplantats 104 geschoben, wodurch dieses in seine Ausgangsposition zurück gedrückt wird. Die Bewegung des zweiten Einführelements 14 erfolgt in Richtung des distalen Endes 34 der Einführvorrichtung 10. Nun kann der erste Teilbereich 118 durch erneutes Zurückziehen des zweiten Einführelements 14 nochmals positioniert werden. Bei einer festgestellten Fehlfunktion kann das Hybridimplantat 100 wieder aus dem Körper entfernt werden. Da Schritt 112 einen alternativen Verfahrensschritt darstellt, ist er in Fig. 11 als Kasten mit gestrichelter Umrandung gezeigt. Wurde eine korrekte Position erreicht, wird das zweite Einführelement 14 in Schritt 214 Fixieren proximal bzw. wieder mit dem Griffsegment 26 am dritten Einführelement 16 oder am Griffsegment 28 fixiert.

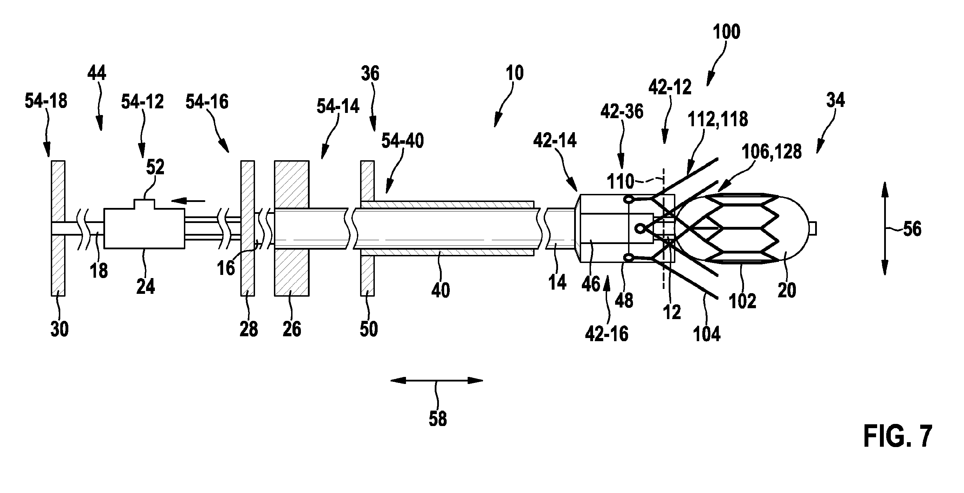

In einem nachfolgenden Schritt 216 Zurückziehen wird nun das fremdexpandierbare Teilimplantat 102 und die Expansionshilfe 20 positioniert. Dabei tritt der Interaktionsbereich 106 des fremdexpandierbaren Teilimplantats102 in den Innendurchmesser Dᵢ des Interaktionsbereichs 112 des selbstexpandierenden Teilimplantats 104 ein. Jedoch erfolgt dies nur soweit, dass der Interaktionsbereich 106 distal von der Klappenebene 110 des Klappeneinsatzes 108 angeordnet ist, wodurch eine Beschädigung des Klappeneinsatzes 108 vermieden wird (siehe Fig. 7, in der die Klappenebene 110 lediglich schematisch durch eine gestrichelte Linie angedeutet ist). Das Zurückziehen erfolgt nach einem Lösen der Verbindung zwischen dem Griffsegment 28 und dem Einführelement 12 indem das erste Einführelement 12 durch Ziehen an dem Griffsegment 30 zusammen mit dem vierten Einführelement 18 in Richtung des proximalen Endes 44 der Einführvorrichtung 10 bewegt wird (siehe Pfeil). Alternativ können das erste und das vierte Einführelement 12, 18 auch über ein Ziehen am Griffsegment 24 bzw. der Weiche 52 bewegt werden. Durch das Bewegen vollziehen das erste Einführelement 12 und das vierte Einführelement 18 eine Relativbewegung in axialer Richtung 58 bezüglich des zweiten und des dritten Einführelemente 14, 16. Somit ist das erste Einführelement 12 verschieblich innerhalb des zweiten Einführelements 14 angeordnet. In der Realität ist ein axialer Abstand zwischen dem Griffsegment 26 und dem Griffsegment 28 größer als hier dargestellt, sodass eine Bewegung des Griffsegments 26 in Richtung des proximalen Endes 44 der Einführvorrichtung 10 zur kompletten Freigabe des zweiten Teilimplantats 104 weiter möglich ist (siehe unten).

Befindet sich nun das erste fremdexpandierbare Teilimplantat 102 in der erwünschten und korrekten Position wird das erste Einführelement 12 in Schritt 114' Fixieren proximal fixiert. Sicherheitshalber werden nochmals in Schritt 208 Kontrollieren die korrekten Positionen des expandierten distalen Teilbereichs 118 des selbstexpandierenden Teilimplantats 104 und die des fremdexpandierbaren Teilimplantats 102 überprüft. Ferner sollte hier erneut in Schritt 210 Testen die Funktion des Klappeneinsatzes 108 überprüft werden.

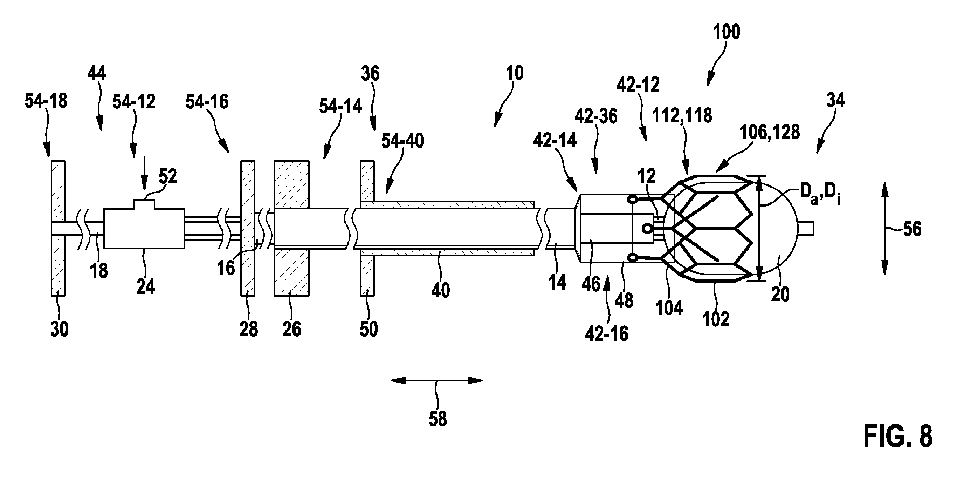

Anschließend wird in einem Schritt 218 Expandieren das vollständige erste fremdexpandierbare Teilimplantat 102 freigesetzt (siehe Fig. 8). Das Expandieren wird hierbei mit Hilfe der Expansionshilfe 20 durchgeführt. Hierzu wird ein nicht näher dargestelltes Medium, wie beispielsweise Druckluft oder Kochsalzlösung, über die Weiche 52 zugeführt (siehe Pfeil), wobei das erste Einführelement 12 hierfür einen nicht näher gezeigten Zuführkanal aufweist. Wie oben schon erwähnt, hat nun der Interaktionsbereich 106 des fremdexpandierbaren Teilimplantats 102 im expandierten Zustand einen Außendurchmesser Dₐ angenommen, der auf den Innendurchmesse Dᵢ des Interaktionsbereichs 112 des selbstexpandierenden Teilimplantats 104 abgestimmt ist (Aus Gründen der besseren Darstellbarkeit ist für beide Durchmesser dieselbe Erstreckung gezeigt, obwohl der Außendurchmesser Dₐ minimal größer ist als der Innendurchmesse Dᵢ). Somit wird durch die Expansion des fremdexpandierbaren Teilimplantats 102 der erste Teilbereich 118 des zweiten selbstexpandierenden Teilimplantats 104 mittels des Interaktionsbereichs 106 des ersten fremdexpandierbaren Teilimplantats 102 an einem Bestimmungsort bzw. dem Implantationsort befestigt (nicht im Detail gezeigt).

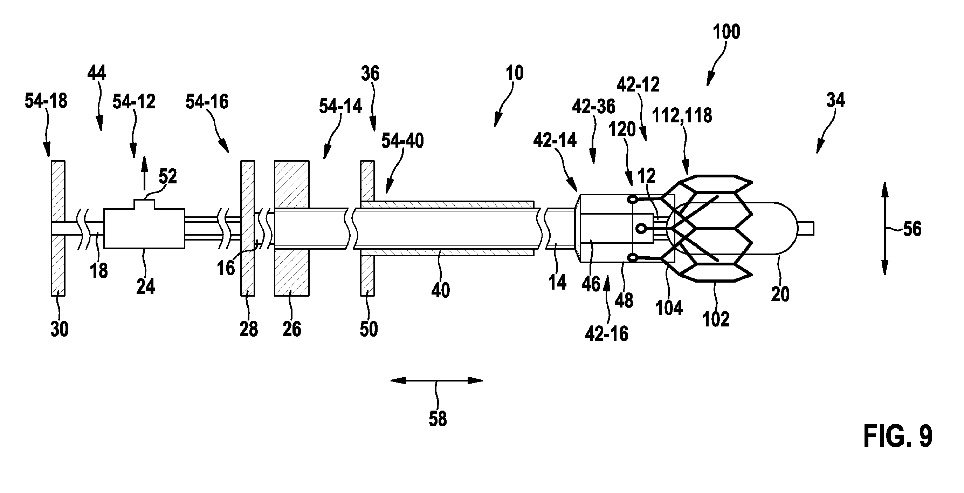

Im nachfolgenden Schritt 220 Entleeren wird nun das Medium aus der Expansionshilfe 20 entfernt (siehe Pfeil), wodurch diese deflatiert. Das fremdexpandierbare Teilimplantat 102 und dadurch auch der Teilbereich 118 des selbstexpandierenden Teilimplantats 104 verbleiben in ihrem expandierten Zustand am Implantationsort (siehe Fig. 9). Erneut erfolgt in Schritt 208 Kontrollieren die Überprüfung der korrekten Positionen des expandierten distalen Teilbereichs 118 des selbstexpandierenden Teilimplantats 104 und die des fremdexpandierbaren Teilimplantats 102.

Im Anschluss wird in Schritt 222 Lösen das selbstexpandierende Teilimplantat 104 vom Implantathalter 46 gelöst Dies erfolgt beispielsweise durch radiales Zurückziehen der Haken des Implantathalters 46 aus den Befestigungsösen 126 des proximalen Endbereichs 120 des selbstexpandierenden Teilimplantats 104 (nicht im Detail gezeigt).

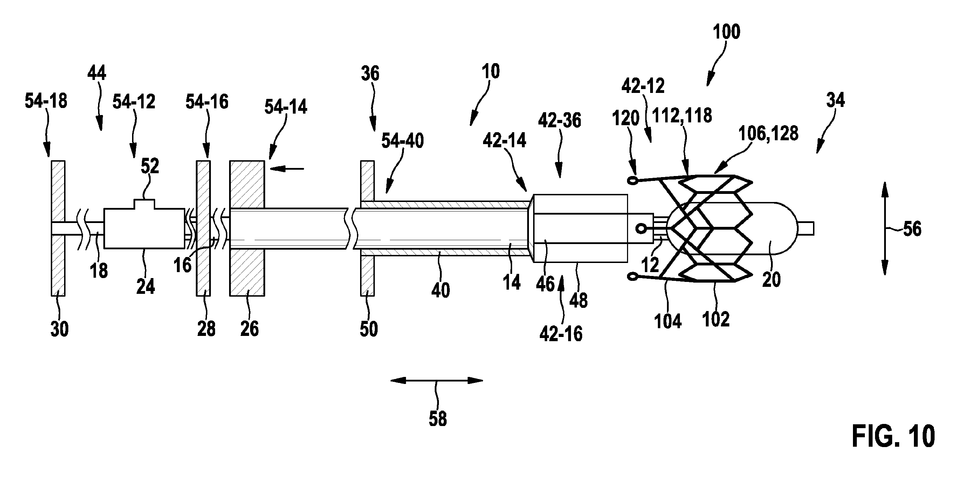

Bei dem nächsten Schritt 224 Teilexpandieren wird ein zweiter Teilbereich 120 bzw. der proximale Endbereichs 120 des zweiten selbstexpandierenden Teilimplantats 104 freigelegt, wodurch dieser selbsttätig expandiert (vgl. Fig. 10). Hierbei wird das zweite Einführelement 14 mit dem Griffsegment 26 nach dem Lösen der Fixierung erneut in Richtung des proximalen Endes 44 der Einführvorrichtung 10 gezogen (siehe Pfeil), wodurch das zweite Einführelement 14 eine Relativbewegung in axiale Richtung 58 bezüglich der anderen drei Einführelemente 12, 16, 18 vollzieht. Das Teilexpandieren des zweiten Teilbereichs 120 wird somit durch eine Relativbewegung des zweiten Einführelements 14 bezüglich des dritten Einführelements 16 ausgelöst. Die Ziehbewegung erfolgt hierbei so lange bis das Hybridimplantat 100 vollständig freigesetzt ist.

Nachfolgend wird in Schritt 226 Schließen die Schutzhülle 48 geschlossen und im Anschluss wird in Schritt 228 Entfernen die Einführvorrichtung 10 zurückgezogen und aus dem Körper entfernt. Das Hybridimplantat 100 verbleibt vollständig positioniert im Körper (nicht gezeigt). Um die erfolgreiche Implantation zu verifizieren wird letztlich in Schritt 230 Untersuchen ein Aortogramm aufgenommen.

Prinzipiell kann eines der Bauteile/Bestandteil der Einführvorrichtung 10 oder des Hybridimplantats 100 auch aus einem röntgensichtbaren Metall, wie beispielsweise Edelstahl, Tantal, Gold oder Platin gebildet sein. Somit könnte anhand eines hier nicht gezeigten Röntgengeräts während der Implantation des Hybridimplantats 100 mittels der Einführvorrichtung 10 ein Fortschritt beispielsweise der Katheterspitze 38 oder eines der Einführelemente 12 14, 16, 18 und damit des Hybridimplantats 10 sowie eine korrekte Position des Hybridimplantats 100 an einem Implantationsort überwacht werden.

### Bezugszeichen

- 10: Einführvorrichtung
- 12: Einführelement
- 14: Einführelement
- 16: Einführelement
- 18: Einführelement
- 20: Expansionshilfe
- 22: Führungselement
- 24: Griffsegment
- 26: Griffsegment
- 28: Griffsegment
- 30: Griffsegment
- 32: Stopp
- 34: Ende
- 36: Schaftbereich
- 38: Katheterspitze
- 40: Stabilisierungsschlauch
- 42: Ende
- 44: Ende
- 46: Implantathalter
- 48: Schutzhülle
- 50: Griffsegment
- 52: Weiche
- 54: Ende
- 56: Richtung
- 58: Richtung
- 100: Hybridimplantat
- 102: Teilimplantat
- 104: Teilimplantat
- 106: Interaktionsbereich
- 108: Klappeneinsatz
- 110: Klappenebene
- 112: Interaktionsbereich
- 114: Zelle
- 116: Dichtstruktur
- 118: Bereich
- 120: Bereich
- 122: Grundgerüst
- 124: Umfangsrichtung
- 126: Befestigungsösen
- 128: Endbereich
- 200: Vordehnen
- 202: Einbringen
- 204: Positionieren
- 206: Teilexpandieren
- 208: Kontrollieren
- 210: Testen
- 212: Repositionieren
- 214: Fixieren
- 216: Zurückziehen
- 218: Expandieren
- 220: Entleeren
- 222: Lösen
- 224: Teilexpandieren
- 226: Schließen
- 228: Entfernen
- 230: Untersuchen
- Da: Außendurchmesser
- Di: Innendurchmesser

## Patentansprüche

1. Einführvorrichtung (10) zum Einführen eines medizinischen Hybridimplantats (100), wobei das Hybridimplantat (100) zumindest zwei Teilimplantate (102, 104) aufweist, von denen zumindest ein erstes Teilimplantat (102) fremdexpandierbar und zumindest ein zweites Teilimplantat (104) selbstexpandierend ausgeführt ist, aufweisend ein erstes Einführelement (12), das zumindest eine Expansionshilfe (20) zum Expandieren des zumindest ersten fremdexpandierbaren Teilimplantats (102) umfasst, und ein zweites Einführelement (14) und zumindest ein drittes Einführelement (16) zur Freigabe des zumindest zweiten selbstexpandierenden Teilimplantats (104), wobei das zumindest erste fremdexpandierbare Teilimplantat (102) mit der Expansionshilfe (20) expandierbar ist und das zumindest zweite selbstexpandierende Teilimplantat (104) durch eine Relativbewegung zwischen dem zweiten Einführelement (14) und dem zumindest dritten Einführelement (16) freisetzbar ist, wobei das erste Einführelement (12) verschieblich innerhalb des zumindest zweiten Einführelements (14) angeordnet ist.

2. Einführvorrichtung nach Anspruch 1, wobei das zumindest dritte Einführelement (16) radial zwischen dem ersten Einführelement (12) und dem zweiten Einführelement (14) angeordnet ist und/oder welches dazu ausgebildet ist, zumindest bei einer Expansion des zweiten selbstexpandierenden Teilimplantats (104) das zweite selbstexpandierende Teilimplantat (104) in Position zu halten.

3. Einführvorrichtung nach Anspruch 1 oder 2, wobei zumindest ein viertes Einführelement (18) vorgesehen ist, welches radial innerhalb des ersten Einführelements (12) angeordnet ist und/oder welches dazu ausgebildet ist, ein Führungselement (22) aufzunehmen.

4. Einführvorrichtung nach einem der vorhergehenden Ansprüche, wobei zumindest ein viertes Einführelement (18) vorgesehen ist und/oder wobei das zweite Einführelement (14) in Bezug auf das zumindest erste Einführelement (12) und/oder in Bezug auf das zumindest vierte Einführelement (18) axial verschieblich angeordnet ist.

5. Einführvorrichtung nach Anspruch 4, wobei das erste Einführelement (12) und/oder das zweite Einführelement (14) und/oder das dritte Einführelement (16) und/oder das zumindest vierte Einführelement (18) jeweils zumindest ein Griffsegment (24, 26, 28, 30) aufweist, und/oder wobei das Griffsegment (24, 30) jeweils dazu ausgebildet ist, in zumindest einem Betriebszustand das erste und das zumindest vierte Einführelement (12, 18) unabhängig von dem zweiten und dem zumindest dritten Einführelement (14, 16) zu bewegen und/oder wobei das Griffsegment (26) dazu ausgebildet ist, in zumindest einem Betriebszustand das zweite Einführelement (14) unabhängig von jeweils dem ersten, dem zumindest dritten und dem zumindest vierten Einführelement (12, 16, 18) zu bewegen.

6. Einführvorrichtung zumindest nach Anspruch 4 oder 5, wobei das zumindest dritte Einführelement (16) einen Stopp (32) aufweist, welcher eine Relativbewegung des zweiten Einführelements (14) bezüglich des zumindest dritten Einführelements (16) limitiert.

7. Einführvorrichtung nach einem der vorhergehenden Ansprüche, wobei im montierten Zustand des Hybridimplantats (100) das zumindest erste fremdexpandierbare Teilimplantat (102) einem distalen Ende (34) der Einführvorrichtung (10) zugewandt ist und/oder das zumindest zweite selbstexpandierende Teilimplantat (104) von dem distalen Ende (34) der Einführvorrichtung (10) abgewandt ist.

8. Einführvorrichtung nach einem der vorhergehenden Ansprüche, wobei das erste fremdexpandierbare Teilimplantat (102) zumindest einen Interaktionsbereich (106) zu einer Interaktion mit dem zumindest zweiten selbstexpandierenden Teilimplantat (104) und das zumindest zweite selbstexpandierende Teilimplantat (104) zumindest einen Klappeneinsatz (108) aufweisen, wobei der zumindest eine Interaktionsbereich (106) des ersten fremdexpandierbaren Teilimplantats (102) infolge einer Relativbewegung zwischen dem ersten Einführelement (12) und dem zweiten Einführelement (14) der Einführvorrichtung (10) distal von einer Klappenebene (110) des Klappeneinsatzes (108) anordenbar ist.

9. Medizinisches Hybridimplantat (100), insbesondere zu einem Einführen mit einer Einführvorrichtung (10) nach zumindest einem der Ansprüche 1 bis 8, aufweisend zumindest zwei Teilimplantate (102, 104), von denen zumindest ein erstes Teilimplantat (102) fremdexpandierbar und zumindest ein zweites Teilimplantat (104) selbstexpandierend ausgeführt ist, wobei zumindest ein Interaktionsbereich (106) des ersten fremdexpandierbaren Teilimplantats (102) im expandierten Zustand einen Außendurchmesser (Dₐ) und zumindest ein Interaktionsbereich (112) des zumindest zweiten selbstexpandierenden Teilimplantats (104) im expandierten Zustand einen Innenmesser (Dᵢ) aufweisen, wobei eine Dimension des Außendurchmessers (Dₐ) des zumindest einen Interaktionsbereichs (106) des ersten fremdexpandierbaren Teilimplantats (102) an eine Dimension des Innendurchmessers (Dᵢ) des zumindest einen Interaktionsbereichs (112) des zumindest zweiten selbstexpandierenden Teilimplantats (104) angepasst ist, so dass bei einer Relativbewegung zwischen einem ersten Einführelement (12) und einem zweiten Einführelement (14) der Einführvorrichtung (10) zumindest der eine Interaktionsbereich (106) des ersten fremdexpandierbaren Teilimplantats (102) in den zumindest einen Interaktionsbereich (112) des zumindest zweiten selbstexpandierenden Teilimplantats (104) eintreten kann und der Interaktionsbereich (112) des zweiten selbstexpandierenden Teilimplantats (104) im expandierten Zustand mittels des Interaktionsbereich (106) des ersten fremdexpandierbaren Teilimplantats (102) an einem Bestimmungsort befestigbar ist.

10. Medizinisches Hybridimplantat nach Anspruch 9, wobei das zumindest zweite selbstexpandierende Teilimplantat (104) zumindest einen Klappeneinsatz (108) aufweist.

11. Medizinisches Hybridimplantat nach Anspruch 9 oder 10, wobei der zumindest eine Interaktionsbereich (106) des ersten fremdexpandierbaren Teilimplantats (102) infolge der Relativbewegung zwischen dem ersten Einführelement (12) und dem zweiten Einführelement (14) der Einführvorrichtung (10) distal von einer Klappenebene (110) des Klappeneinsatzes (108) anordenbar ist.

12. Medizinisches Hybridimplantat nach zumindest einem der Ansprüche 9 bis 11, wobei das zumindest zweite selbstexpandierende Teilimplantat (104) eine Anzahl von Zellen (114, 114') aufweist, wobei die Anzahl so ausgewählt ist, dass das zweite selbstexpandierende Teilimplantat (104) eine geringe Radialkraft aufweist.

13. Medizinisches Hybridimplantat nach zumindest einem der Ansprüche 9 bis 12, wobei das zumindest erste fremdexpandierbare Teilimplantat (102) zumindest eine Dichtstruktur (116) aufweist, die dazu ausgebildet ist, im implantierten Zustand eine Leckage zu reduzieren.

14. Verfahren zu einem Einführen eines medizinischen Hybridimplantats (100), insbesondere nach zumindest einem der Ansprüche 9 bis 13, mit Hilfe einer Einführvorrichtung (10), insbesondere nach einem der Ansprüche 1 bis 8, aufweisend zumindest die folgenden Schritte:
- Teilexpandieren (206) eines ersten Teilbereichs (118) eines zumindest zweiten selbstexpandierenden Teilimplantats (104), insbesondere im auf der Einführvorrichtung 10 montierten Zustand des zumindest zweiten selbstexpandierenden Teilimplantats 104 eines distalen Endbereichs (118) des zumindest zweiten selbstexpandierenden Teilimplantats (104);
- Expandieren (218) eines vollständigen zumindest ersten fremdexpandierbaren Teilimplantats (102);
- Teilexpandieren (224) eines zweiten Teilbereichs (120) des zumindest zweiten selbstexpandierenden Teilimplantats (104), insbesondere im auf der Einführvorrichtung 10 montierten Zustand des zumindest zweiten selbstexpandierenden Teilim plantats 104 eines proximalen Endbereichs (120) des zumindest zweiten selbstex pandierenden Teilimplantats (104).

15. Verfahren nach Anspruch 14, wobei
- das Teilexpandieren (206) des ersten Teilbereichs (118) des zumindest zweiten selbstexpandierenden Teilimplantats (104) durch eine Relativbewegung eines zweiten Einführelements (14) bezüglich eines dritten Einführelements (16) der Einführvorrichtung (10) ausgelöst wird, wobei die Relativbewegung anhand eines Stopps (32) eines zumindest dritten Einführelements (16) limitiert wird;
- das Expandieren (218) des ersten fremdexpandierbare Teilimplantats (102) mit Hilfe zumindest einer Expansionshilfe (20) durchgeführt wird, wodurch der erste Teilbereich (118) des zweiten selbstexpandierenden Teilimplantats (104) mittels eines Interaktionsbereichs (106) des ersten fremdexpandierbaren Teilimplantats (102) an einem Bestimmungsort befestigt wird;
- das Teilexpandieren (224) des zweiten Teilbereichs (120) des zumindest zweiten selbstexpandierenden Teilimplantats (104) durch eine Relativbewegung des zweiten Einführelements (14) bezüglich des dritten Einführelements (16) der Einführvorrichtung (10) ausgelöst wird, wodurch das Hybridimplantat (100) vollständig freigesetzt wird.
